# EUROPEAN PATENT SPECIFICATION

(11) **EP 4 633 548 B1**
(45) Date of publication and mention of the grant of the patent: **25.11.2026**
(21) Application number: 23817452.8
(22) Date of filing: 04.12.2023
(51) Int. Cl.: A61F 2/89, A61F 2/07, A61F 2/915

(54) **IMPLANT AND COVERING FOR AN IMPLANT**
IMPLANTAT UND ABDECKUNG FÜR EIN IMPLANTAT
IMPLANT ET REVÊTEMENT POUR IMPLANT

(30) Priority: 15.12.2022 EP 22213860
(43) Date of publication of application: 22.10.2025
(73) Proprietor: Cortronik GmbH, 18119 Rostock-Warnemünde (DE)
(72) Inventor: KLEMM, Ralf, 18209 Parkentin (DE); THOEBE, Martin, 18209 Bartenshagen-Parkentin (DE); KUEHN, Johannes, 18057 Rostock (DE)
(74) Representative: WSL Patentanwälte Partnerschaft mbB
(86) International application number: PCT/EP2023/084094
(87) International publication number: WO 2024/126123

(56) References cited:
- DE-A1- 102007 032 340
- US-A1- 2003 204 247
- US-A1- 2006 259 125
- US-B1- 6 287 335
- US-B1- 6 626 938

## Description

The present invention relates to an implant being an endovascular prosthesis, heart valve or implant in the neurovascular field having a body which forms a circumferential and perforated lateral surface, wherein the body may assume a compressed state and an expanded state. The implant further comprises, on the lateral surface, a covering which is also referred to as a cover or skirt. The invention also relates to a method for producing such an implant.

Graft membranes are known, for example from US 2003/0204247 A1 and US 6,287,335 B1. Further, implants with a body (main body, support body, framework), which forms a circumferential and perforated lateral surface, and with a covering (cover, skirt) are known, as well. US 6626938 B1 discloses a stent graft having a radially expandable stent and a graft covering provided with longitudinal pleats to facilitate crimping and deployment of the stent graft.

In particular, implants in the form of endovascular prostheses (endoprostheses, stents, stent grafts) may be used to treat stenoses (vasoconstrictions). They usually have a hollow-cylindrical or tubular body that is open at both longitudinal ends of the tubes. Such a body is often formed predominantly from a number of interconnected struts, which may be at least partially zigzag-shaped or meander-shaped. The implant is placed in the vessel to be treated and serves to support the vessel. The covering arranged on the body may prevent restenosis in this case, as the material of the covering prevents tissue from growing into the interior of the stent. In addition, such stents (covered stents) are used when the vessel to be treated has a wall injury (dissection). If the stent is used immediately as an emergency treatment for a dissection-related occlusion or vessel rupture, the procedure is also called bail-out stenting. Such implants are also used for bypass surgery. Implants such as prosthetic heart valves also have a stent-like body which is provided with a covering (skirt), at least in portions, in order to prevent regurgitation. The invention may also be used for stents and similar implants in the neurovascular field.

The body of such implant has two states, namely a compressed state and an expanded state. In particular, the body has a compressed state or an expanded state, and the body is configured so to be able to transition from the compressed state to the expanded state and vice versa. In the compressed state of the body with the smaller outer diameter, the implant may be transported through the vascular system to the treatment site, for example by means of a catheter. There, the implant is dilated, for example by means of a balloon of a catheter, and transferred to the expanded state in which the implant has a larger outer diameter. The transition to the expanded state may also occur due to a transformation of a shape memory alloy by temperature change. The implant with the expanded body then remains at the respective treatment site in the patient and continues to develop its desired effect there.

The transition from the compressed state to the expanded state (or vice versa) involves a considerable increase in the diameter of the body. When using a covering placed on the lateral surface of the body, the covering must allow for this change in diameter in such a way that the covering is not damaged or the compression or expansion of the body is not hindered. This places special requirements on the arrangement and construction of the covering.

It is known to use electrostatic forces to spin polyurethane fibres onto the lateral surface of an expanded stent, where they form a thin and highly elastic membrane. On the one hand, this creates an implant with a very small outer diameter in the compressed state, and on the other hand, such an implant may be easily compressed (for example by means of crimping), since the material of the spun covering compresses when the diameter is reduced and yields into the remaining gaps between the struts of the body. Such a spun covering is costly to produce and is also limited to materials that allow spinning as well as stand up when compressed. Alternatively, a sheathing with an overlapping region that is formed in the compressed state may be provided. In this variant, the covering is unevenly stressed during expansion, namely in particular in the region of the overlap. Furthermore, known sheathings show that, due to their folding structure, they transmit a torque to the expanding implant along its longitudinal axis during unfolding. Such a torque may lead to torsion of the implant and make it difficult or impossible for the implant to expand. Therefore, especially for non-expandable or low-expandable covering materials or materials that do not stand up, implant solutions are sought that allow for easy and uniform compression or expansion along the entire body.

The above problem is solved by an implant (endovascular prosthesis, heart valve or implant in the neurovascular field) having the features of claim 1, and a method for producing such implant having the features of claim 8.

In particular, the above problem is solved by an implant being an endovascular prosthesis, heart valve or implant in the neurovascular field comprising a body forming a circumferential and perforated lateral surface, wherein the body has a compressed state and is configured to transition to an expanded state, wherein the lateral surface in the compressed state of the body is covered at least in portions with a covering, wherein a tubular covering for an implant, in which the covering is laid along a circumferential direction of the tube in a zigzag folding.

The tubular covering has an even number of zigzag foldings lying next to one another in the circumferential direction, for example the covering has a first zigzag folding and a second zigzag folding or a first zigzag folding, a second zigzag folding, a third zigzag folding and a fourth zigzag folding or a number of 2 x N zigzag foldings, wherein N >= 3, wherein all zigzag foldings are arranged next to one another in the circumferential direction, and wherein in each case two zigzag foldings adjacent in the circumferential direction are in each case folded mirror-symmetrically with respect to the imaginary mirror plane arranged between the respective two zigzag foldings.

The covering is made of a covering element which is shaped in a tube-like manner and the material of which has a low plastic and/or elastic deformability, but may be bent/folded. In one exemplary embodiment, the length of the tube in the longitudinal direction is selected so that it corresponds to the length of the covering in the expanded state of the implant or body.

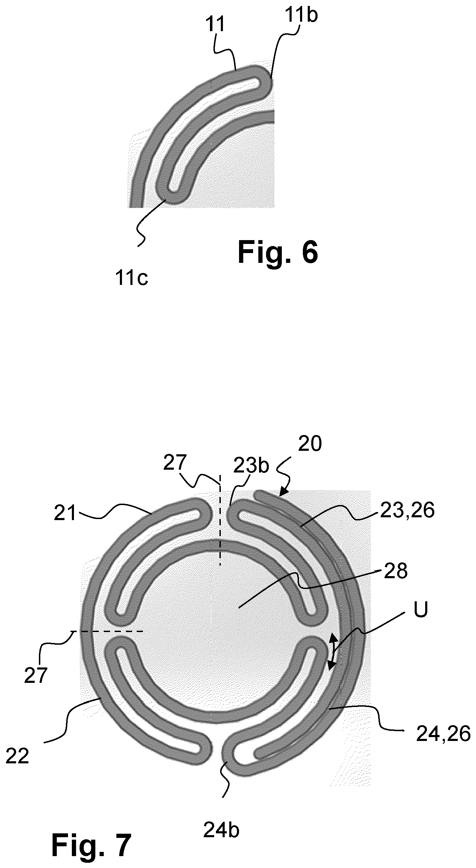

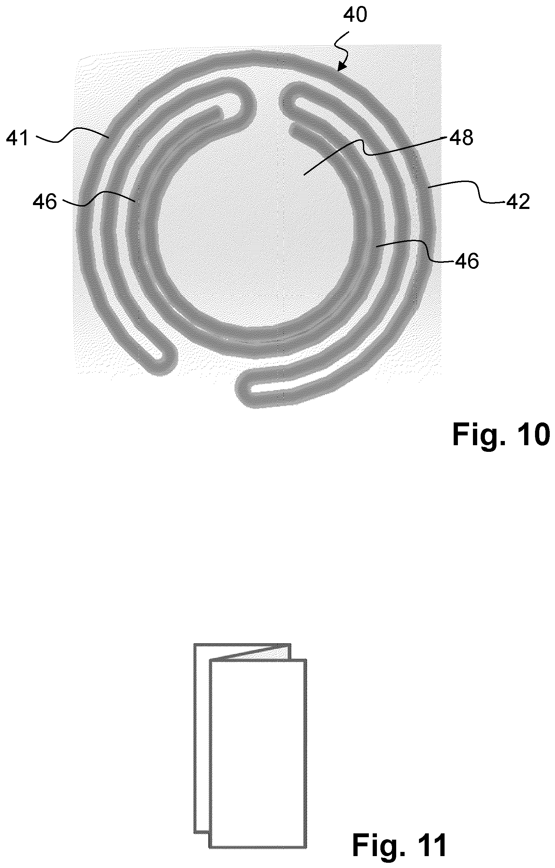

Zigzag folding, Z-folding or leporello folding is the term used to describe a folding in which layers of a sheet-like element or, as in the present case, a tubular element form 3 layers arranged one above the other by two oppositely directed folds which, when viewed in cross-section, form a shape comparable to a Z-shape (cf. Fig. 11), wherein, when applied to the covering, the central slope of the "Z" tends to be parallel to the horizontal portions of the "Z". In other words, the element is first folded in a first direction so that the material is subsequently circumferential again, and then folded in a second direction which is circumferentially opposite the first direction (i.e. zigzag folded). The result of a single zigzag folding is three circumferentially running superimposed layers of the material of the covering and two fold edges oriented in opposite circumferential directions. In cross-section, the zigzag folding is shown in Fig. 6 and described below. The zigzag folding is folded longitudinally along the entire length of the covering tube so that the material of the covering tube is 3-ply in the region of a single zigzag folding along the entire length of the tube.

The covering has a first zigzag folding and at least one second zigzag folding, i.e. at least two zigzag foldings. These are arranged side by side along the circumferential direction. This means that each zigzag folding is formed in a separate portion of the covering along the circumferential direction, so that only three layers of the covering material are superimposed in each case. Further, two adjacent zigzag foldings are provided in such a way that they are folded mirror symmetrically with respect to an imaginary mirror plane, which runs transversely to the circumferential direction, between the respective two adjacent zigzag foldings and in the longitudinal direction of the tube. This means that, in relation to the radial direction, the fold edges of adjacent zigzag foldings lying on the same plane point in opposite directions. Opposite fold edges of adjacent zigzag foldings form openings that facilitate unfolding of the covering during the transition to the expanded state. The foldings of two adjacent zigzag foldings run in opposite directions, wherein the lower, middle and upper layers of adjacent zigzag foldings each lying substantially on the same plane with respect to the radial direction. It should be noted here that the arrangement of the covering material of adjacent zigzag foldings is not perfectly mirror symmetrical to the imaginary mirror plane, but may be described as similar (length of the folds, structure of the folds and the like). The mirror symmetry of adjacent zigzag foldings defined above is understood in relation to the folding (i.e. flipping) of the covering material.

The covering folded as shown above is assembled with the compressed body before or after folding to complete the implant, i.e. the compressed body is placed in an inner free space created by folding and applying the folds in the circumferential direction of the covering. This produces an easily expandable implant with a comparatively small outer diameter in the compressed state, in which the covering may have a low extensibility. For example, a pericardial covering may be used which has a low tendency to thrombus. Due to the symmetrical arrangement of the folding or the symmetrical folding technique in the form of the zigzag folding, the tensile forces applied when the covering is unfolded at the treatment site during expansion of the implant or body are distributed over the zigzag foldings arranged next to each other, so that the tensile force is evenly distributed around the entire circumference of the implant or covering. Tensile force peaks are thus avoided. As a result, less force is required during expansion, which leads to better executability of the expansion. In particular, when using four or six zigzag foldings along the circumference, the distribution of the force is even along the entire circumference of the covering.

In one exemplary embodiment, as described above, the first zigzag folding and the at least one second zigzag folding are placed such that the covering forms an inner, substantially cylindrical free space. In this way, the folds of the covering lie closely together along the circumferential direction and the insertion of the compressed body of the implant into the free space is facilitated. This also prevents damage to the cover when the body is inserted into the free space.

In one exemplary embodiment, the covering has a connecting portion in which a first layer of the covering material and a second layer of the covering material are superimposed and attached to each other along the longitudinal direction of the covering. Alternatively, the covering material may be manufactured as a tube and therefore have no connecting portion, for example in a corresponding shaping process for a plastics material or by means of a weaving, spinning or knitting process. If the covering is made from a sheet-like or film piece-like material, then the tubular covering element is made from this material, for example, in such a way that two end portions which have the length of the desired covering element are placed one on top of the other and joined or fastened to each other. The portion where the end portions lie on top of each other is called the connecting portion. The connecting portion extends here along the entire length of the covering element. The end portions lying on top of each other may be fastened by means of one or more seams, an adhesive joint or a seamless joining technique by means of chemical cross-linking, which is described, for example, in WO 2022/090417 A1. This enables a simple production of the covering element.

The covering material may have a single layer or may be multi-layered. The covering material may comprise one or more materials from the group comprising biocompatible plastics, for example polymers from the group of cellulose, collagen, albumin, casein, polysaccharides (PSAC), polylactide (PLA), poly-L-lactide (PLLA), polyglycol (PGA), poly-D,L-lactide-co-glycolide (PDLLA-PGA), polyhydroxybutyric acid (PHB), polyhydroxyvaleric acid (PHV), polyalkylcarbonates, polyorthoesters, polyethylene terephthalate (PET), polymalonic acid (PML), polyanhydrides, polyphosphazenes, polyamino acids and their copolymers, hyaluronic acid, in this case, depending on the desired properties, in pure form, in derivatised form, in the form of blends or as copolymers, biodegradable material, bioresorbable material, biological material, for example natively dried tissue, for example pericardium, or may consist of one or more materials from this group.

The body may consist of or contain biocompatible materials and/or biodegradable materials, wherein the material comprises at least one material selected from the group comprising metallic biodegradable materials, in particular based on magnesium or a magnesium alloy, for example WE43, magnesium-zinc-aluminium, magnesium-aluminium or magnesium-zinc-calcium, shape-memory alloys, for example nitinol, and the biodegradable plastics indicated above.

Biodegradation is understood to mean hydrolytic, enzymatic and other metabolic degradation processes in the living organism, which are mainly caused by the body fluids coming into contact with the implant and lead to a gradual dissolution of at least large parts of the body or the implant. The term biocorrosion is often used synonymously with the term biodegradation. The term bioresorption includes the subsequent resorption of the degradation products by the living organism. The aim of using biodegradable implants is that they are degraded by the organism at a point in time when they are no longer needed, for example, in terms of their supportive effect, and consequently are not present as foreign bodies in the organism any longer than necessary.

In one exemplary embodiment of the covering in which the covering element comprises a connecting portion, the connecting portion may be circumferentially adjacent to the folding portion of the covering comprising the first zigzag folding and the at least one second zigzag folding such that the connecting portion and the folding portion do not overlap. In the region of the connecting portion, only two superimposed layers of the covering material are present, whereas in the region of the zigzag foldings, three superimposed layers of the covering material are arranged. This makes the outer diameter of the implant with the covering comparatively small.

In another exemplary embodiment of the covering with a connecting portion, the connecting portion overlaps with at least one fold formed by the first zigzag folding and/or the at least one second zigzag folding. This means that the connecting portion is part of one zigzag folding or two adjacent zigzag foldings. In this case, however, the fold edge is not arranged in the region of the connecting portion, but extends in the circumferential direction next to the connecting portion. With respect to this exemplary embodiment, the connecting portion may be arranged at least partially below the at least one fold formed by the first zigzag folding and/or the at least one second zigzag folding with respect to the radial direction of the covering. This means that the connecting portion is arranged on the inner side of a fold of the first zigzag folding and/or a fold of a second zigzag folding (with respect to the radial direction). Alternatively, the connecting portion may at least partially externally surround the at least one fold formed by the first zigzag folding and/or the at least one second zigzag folding with respect to the radial direction of the covering. In this exemplary embodiment, the connecting portion may be arranged on the outer side of a fold of the first zigzag folding and/or a fold of a second zigzag folding (with respect to the radial direction). In these exemplary embodiments, the connecting portion is integrated into the folding so that it is more uniformly loaded in its behaviour during expansion of the covering. However, the arrangement of the connecting portion in the region of the zigzag folding increases the number of superimposed layers of the covering material, so that the outer diameter of the implant is increased compared to the variant in which the connecting portion is arranged next to the zigzag foldings in the circumferential direction.

The above problem is solved by an implant being an endovascular prosthesis, heart valve or implant in the neurovascular field having a body forming a circumferential and perforated lateral surface, wherein the body has a compressed state and is configured to transition to an expanded state, wherein the lateral surface in the compressed state of the body is at least partially covered with a covering as described above. Such an implant may be, for example, a stent, stent-graft, bail-out stent, a stent portion of a prosthetic heart valve. In one exemplary embodiment, the body is composed of a plurality of interconnected struts which form a substantially hollow-cylindrical shape when compressed. In principle, such bodies have already been described in detail in various variants, so that they will not be discussed in greater detail below. For example, the struts may form annular portions composed of cells (meshes) formed by a plurality of struts (ribs, bars), for example struts forming zigzag-shaped or meander-shaped structures. Here, two or more than two struts may meet in a so-called node. The ring-shaped portions may be connected to each other by further struts.

In particular, the body of the implant has two states, namely a compressed state and an expanded state. In particular, the body has a compressed state or an expanded state, and the body is configured to be able to transition from the compressed state to the expanded state and vice versa.

The body assumes at least two states, namely a compressed state with a small outer diameter and an expanded state with a larger outer diameter. In particular, the body has a compressed state or an expanded state, and the body is configured to be able to transition from the compressed state to the expanded state and vice versa. In the compressed state, the catheter may be used to guide the implant through narrow vessels into the vessel to be treated and positioned at the treatment site. The transition from the compressed state to the expanded state is made by expanding the structure of the body, for example by means of a balloon. The expansion of the supporting structure causes the stent to unfold. Alternatively, the transition to the expanded state in a self-expanding body occurs when the transformation temperature is exceeded.

In the present disclosure, the term "vessel" is used to refer to all vessels, organs or other body cavities of the patient into which a generic implant may be inserted for treatment.

Further, the above problem is solved by a method for producing an implant being an endovascular prosthesis, heart valve or implant in the neurovascular field as described above, said method comprising the following steps:
- providing an unfolded tubular covering element having a predetermined length in the longitudinal direction,
- folding the covering element for producing the covering in such a way that the material is laid along the circumferential direction in a first zigzag folding and at least one second zigzag folding, which are arranged next to one another in the circumferential direction and are configured in such a way in that in each case two, in the circumferential direction, adjacent zigzag foldings of the first zigzag folding and of the at least one second zigzag folding are folded mirror-symmetrically with respect to an imaginary mirror plane which runs transversely with respect to the circumferential direction, between the two adjacent zigzag foldings and in the longitudinal direction of the tube.

This is a particularly simple production process for the implant with the covering explained in detail above. This has the advantages outlined above.

In one exemplary embodiment, the folded covering may be fixed by means of a fixing bath so that it is dimensionally stable and retains the shape and/or folding even when the implant is produced.

As already described above, in one exemplary embodiment, the unfolded tubular covering element may be produced by arranging two end portions of a flat covering element one above the other and firmly connecting these superimposed end portions along the longitudinal direction of the tube. Alternatively, the covering element is produced directly with a tubular form.

In the method for producing above-described implant, the following steps are carried out:
- providing a body in a compressed state,
- arranging the compressed body within an inner free space of the folded covering element or the unfolded covering element.

In one exemplary embodiment, after folding the covering element, the body may be arranged within the free space of this element. These separately performed process steps (producing the folded covering and mounting it on the compressed, for example crimped body) may ensure that the properties of the balloon of a balloon catheter, on which the implant is then placed for transport to the treatment site in the patient, are not affected by chemicals of the fixing bath. Alternatively, the folding and, if necessary, fixing may be carried out when the compressed body is already located within the inner free space of the tubular covering element.

The invention is explained below on the basis of an exemplary embodiment and with reference to the figures.

The figures show schematically:
- Fig. 1: a first exemplary embodiment of an implant in an expanded state in a perspective view from the side,
- Fig. 2: an exemplary embodiment of a body for an implant in the expanded state in a perspective view from the side,
- Fig. 3: the body according to Fig. 2 in a compressed state in a perspective view from the side,
- Fig. 4: an exemplary embodiment of a covering element before the folding in a perspective view from the side,
- Fig. 5: a first exemplary embodiment of a covering in a perspective view from the side,
- Fig. 6: a portion of the covering according to Fig. 5 in a view from the front,
- Fig. 7: a second exemplary embodiment of a covering in a view from the front,
- Fig. 8: the exemplary embodiment of the covering according to Fig. 7 in a perspective view from the side,
- Fig. 9: a third exemplary embodiment of a covering in a perspective view from the side,
- Fig. 10: a fourth exemplary embodiment of a covering in a view from the front, and
- Fig. 11: the zigzag folding in a perspective view from the side.

The invention is explained below on the basis of a covered stent. It may also be used in the same or similar way for other implants mentioned above.

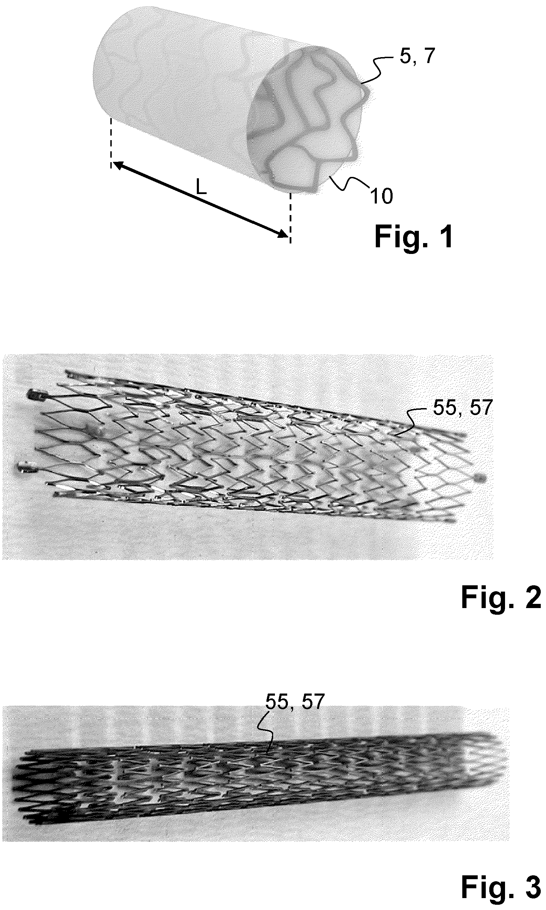

Fig. 1 shows an implant in the form of a covered, hollow-cylindrical stent with a body 5 consisting of a plurality of struts 7 and with a covering 10 in an expanded state. The covering 10 is arranged on the perforated lateral surface of the body 5. The covering 10 has a length L in the longitudinal direction which is slightly smaller than the length of the body 10.

By way of example, Fig. 2 and 3 show a further example of a hollow cylindrical body 55 in an expanded state (Fig. 2) and in a compressed state (Fig. 3). It is clear from Fig. 1 to 3 that the body 5, 55 is composed, in a known manner, of portions with struts 7, 57 arranged in a zigzag or meander shape.

The coverings explained below are suitable in the configuration shown in each case, in particular in the folding shown, to be mounted on the body 5, 55 in the compressed state. The configuration is particularly suitable for a low-stretch covering material (for example pericardium) which is used because it has good properties, for example with regard to biocompatibility. An implant with a covering configured as shown and applied to the compressed body may be expanded with low force due to the symmetrical folding, because the occurring forces are distributed over the symmetrically arranged and configured folding portions.

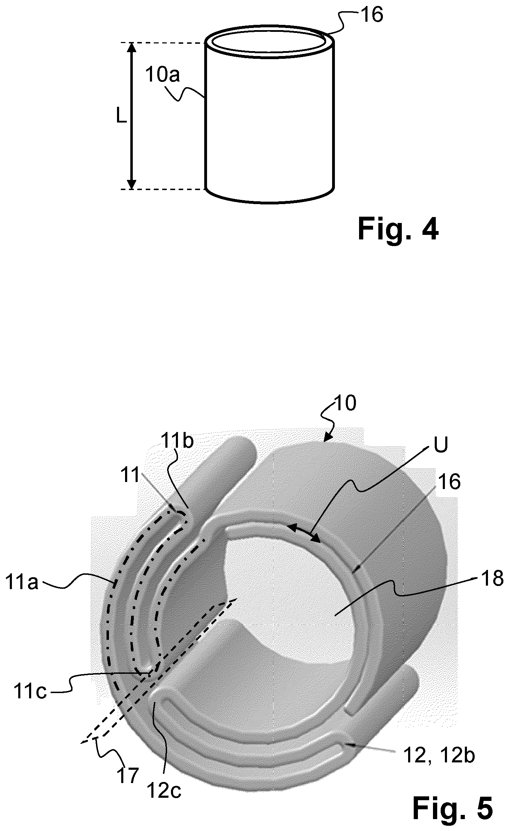

To produce the covering, an unfolded covering element 10a shown in Fig. 4 is first provided. The covering element 10a is, for example, a pericardial tube with a connecting portion 16. The connecting portion 16 is formed by superimposing and firmly connecting two end portions of a sheet-like pericardial piece, for example by means of cross-linking. The connecting portion 16 extends along the entire length L of the covering element 10a.

Alternatively, the folding of the covering element 10a may also be performed during the production of the covering element 10a, for example by an embossing process.

Subsequently, the covering element 10a is folded along the circumferential direction U in such a way that two zigzag foldings 11, 12 are formed, which are arranged in the manner shown in Fig. 5. For each zigzag folding 11, 12, the wall of the covering element 10a is folded twice, first in one direction and then in the opposite direction (with respect to the circumferential direction). The first zigzag folding 11 is marked by a dash-and-dot line 11a in Fig. 5 for better illustration, and is shown separately in Fig. 6 in a front view (also corresponding to the cross-section). Each zigzag folding has two fold edges, which are marked 11b and 11c in respect of the first zigzag folding 11. The fold edges 11b, 11c are oriented in opposite directions along the circumferential direction U, i.e. they are closed or opened in opposite directions. Each zigzag folding 11, 12 has three superimposed layers of covering material in the respective folding portion. In practice, unlike as shown in the figures, these lie directly on top of each other so that they are in contact with one another. The spaced representation is provided here for better illustration.

The first zigzag folding 11 and the second zigzag folding 12 are arranged next to each other in the covering 10 in the circumferential direction U, along the entire length L and in such a way that the folding of the second zigzag folding 12 is mirror-symmetrical to the folding of the first zigzag folding 11 with respect to an imaginary mirror plane 17. The imaginary mirror plane runs transversely to the circumferential direction U, between the first zigzag folding 11 and the second zigzag folding 12 and along the entire length L of the covering 10. In the region of the mirror plane 17, an opening is formed between the fold edge 11c of the first zigzag folding 11 and the fold edge 12c of the second zigzag folding 12, which facilitates the unfolding of the covering 10.

The connecting portion 16 is also arranged in such a way that it does not overlap with the first zigzag folding 11 or with the second zigzag folding 12 in the circumferential direction U. In this way, a particularly small outer diameter of the covering 10 may be achieved, as a maximum of three layers of covering material lie on top of each other in the region of the zigzag foldings 11, 12.

After the corresponding folding, the covering 10 may be fixed by means of a fixing bath. Subsequently, a compressed body 5, 55 is placed in the inner free space 18 of the covering 10, or the covering 10 is mounted on the perforated lateral surface of the compressed body 5, 55. Thus, the implant is prepared and ready for insertion into a patient. For insertion, the implant may be mounted on a catheter, for example on a balloon of a catheter. For the intended use, the covered stent with the covering 10 is transported by means of the catheter to the site to be treated in a vessel of the patient. There, for example by inflation of the balloon, the body 5, 55 of the stent is expanded/dilated and thereby also the covering 10 is unfolded, so that after completion of the expansion process it is arranged on the body 5 as shown in Fig. 1. Due to the mirror-symmetrical arrangement of the first zigzag folding 11 and the second zigzag folding 12, only a small force is required to unfold the covering 10, which is also distributed evenly over both zigzag foldings 11, 12. Tensile forces therefore do not occur at specific points, so that the unfolding takes place evenly.

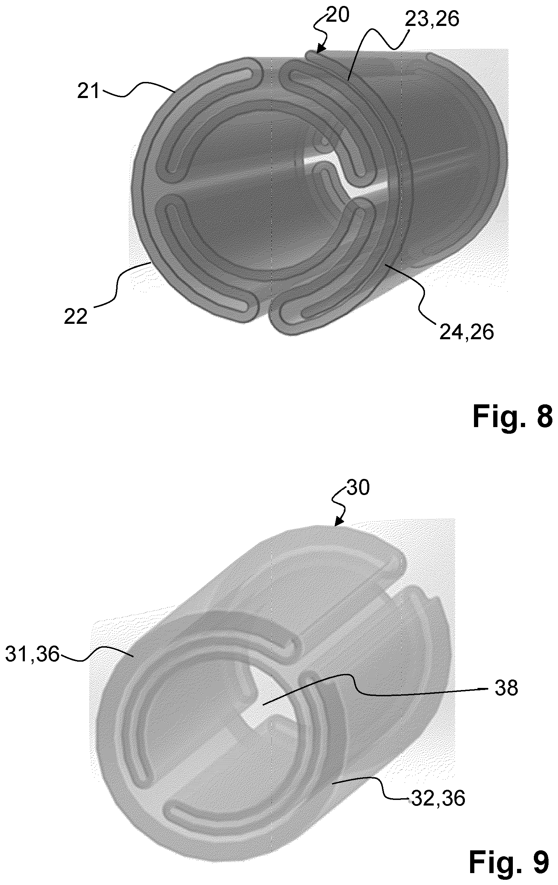

The second exemplary embodiment of a covering 20 shown in Figs. 7 and 8 has a total of four zigzag foldings 21, 22, 23 and 24, which are arranged next to each other along the circumferential direction U. In each case, two adjacent zigzag foldings 21, 22; 22, 24; 24, 23; 23,21 are folded in mirror symmetry analogously to the zigzag foldings 11, 12 of the first exemplary embodiment of the covering. Two imaginary mirror planes 27 are shown by way of example in Fig. 7. Moreover, in this exemplary embodiment, the connecting portion 26 overlaps with two zigzag foldings 23, 24, wherein the fold edges 23b, 24b of the zigzag foldings 23, 24 are arranged to be circumferentially adjacent to the connecting portion 26. This is advantageous because the two-ply connecting portion 26 is less flexible than the (single-ply) covering material. The unfolding of the covering 20 may be even more symmetrical than in the first exemplary embodiment of the covering 10, as the tensile forces are even more evenly distributed over the entire circumference. However, this exemplary embodiment of the covering 20 has a larger circumference, since in the region of the zigzag foldings 23, 24 the covering material has four layers. In addition, this exemplary embodiment is well suited for mechanisation or automation of the mounting of the covering 20.

Analogously to the first exemplary embodiment, the exemplary embodiments for a covering 30, 40 shown in Fig. 9 and 10 each have two zigzag foldings 31, 32 or 41, 42 arranged next to each other in the circumferential direction. The connecting portion 36, 46 overlaps with both zigzag foldings 31, 32 and 41, 42 respectively, wherein in the third exemplary embodiment of the covering 30 shown in Fig. 9 the connecting portion 36 forms the outer part of the zigzag foldings 31, 32 as viewed in the radial direction, while in the fourth exemplary embodiment shown in Fig. 10 the connecting portion 46 forms an inner part of the zigzag foldings 41, 42. In the third exemplary embodiment of the covering 30, the connecting portion 36 clings to the zigzag foldings 31, 32 and therefore forms a protection of these folds, for example during mounting with the body. By contrast, the folds of the zigzag foldings 41, 42 surround the connecting portion 46 as in a flower bud, which has the advantage of better feedability and better crossing properties.

The fourth exemplary embodiment of a covering 40 is very similar to the first exemplary embodiment shown in Fig. 5, because, compared to the latter, the folds in the circumferential direction U are longer in the exemplary embodiment of Fig. 10.

In all exemplary embodiments of the covering 20, 30, 40, the compressed body 5, 55 is mounted in the respective free space inside the covering 20, 30, 40 analogously to the first exemplary embodiment and expanded after being arranged at the treatment site. In this process, the covering 20, 30, 40 unfolds analogously to the first exemplary embodiment.

The body 5, 55 of the implant may, for example, consist of nitinol or of a cobalt-chromium alloy, which changes to the expanded state when a transformation temperature is exceeded. The covering 10, 20, 30, 40 consists for example of pericardium.

## Claims

1. An implant being an endovascular prosthesis, heart valve or implant in the neurovascular field comprising a body (5, 55) forming a circumferential and perforated lateral surface, wherein the body (5, 55) has a compressed state and is configured to transition to an expanded state, wherein the lateral surface in the compressed state of the body (5, 55) is covered at least in portions with a tubular covering (10, 20, 30, 40), **characterized in that** the covering (10, 20, 30, 40) is laid along a circumferential direction (U) of the tubular covering in a first zigzag folding (11, 21, 31, 41) and a second zigzag folding (12, 22, 23, 24, 32, 42) or a first zigzag folding (21), a second zigzag folding (22), a third zigzag folding (23) and a fourth zigzag folding (24) or a number of 2 x N zigzag foldings, wherein N >= 3, wherein all the zigzag foldings are arranged next to one another in the circumferential direction (U) and each zigzag folding has three circumferentially running superimposed layers of the covering material and two fold edges oriented in opposite circumferential directions, wherein in each case two zigzag foldings adjacent in the circumferential direction (U) are in each case folded mirror-symmetrically with respect to the imaginary mirror plane (17, 27) arranged between the respective two adjacent zigzag foldings and in the longitudinal direction of the tube.

2. The implant according to claim 1, **characterised in that** the covering comprises a connecting portion in which a first layer of the covering material and a second layer of the covering material are superimposed and attached to each other along the longitudinal direction of the covering.

3. The implant according to claim 2, **characterised in that** the connecting portion is arranged circumferentially adjacent to the folding portion of the covering comprising the first zigzag folding (11) and the at least one second zigzag folding (12) such that the connecting portion and the folding portion do not overlap.

4. The implant according to claim 2, **characterised in that** the connecting portion overlaps with at least one fold formed by the first zigzag folding (21, 31, 41) and/or the at least one second zigzag folding (22, 23, 24, 32, 42).

5. The implant according to any one of claims 2 and 4, **characterised in that** the connecting portion is arranged at least partially below the at least one fold formed by the first zigzag folding (41) and/or the at least one second zigzag folding (42) with respect to the radial direction of the covering.

6. The implant according to any one of claims 2 and 4, **characterized in that** the connecting portion at least partially externally surrounds the at least one fold formed by the first zigzag folding (21, 31) and/or the at least one second zigzag folding (22, 23, 24, 32) with respect to the radial direction of the covering.

7. The implant according to claim 1, **characterised in that** the body (5, 55) is composed of a plurality of interconnected struts (7, 57) which form a substantially hollow-cylindrical shape when compressed.

8. A method for producing an implant being an endovascular prosthesis, heart valve or implant in the neurovascular field according to any one of claims 1 to 7 said method comprising the following steps:
• providing a body (5, 55) in the compressed state,
• providing an unfolded tubular covering (10a) having a predetermined length (L) in the longitudinal direction,
• folding the covering in such a way that the material is laid along the circumferential direction (U) in a first zigzag folding (11, 21, 31, 41) and a second zigzag folding (12, 22, 23, 24, 32, 42) or a first zigzag folding (21), a second zigzag folding (22), a third zigzag folding (23) and a fourth zigzag folding (24) or a number of 2 x N zigzag foldings, wherein N >= 3, wherein all the zigzag foldings are arranged next to one another in the circumferential direction (U) and each zigzag folding has three circumferentially running superimposed layers of the covering material and two fold edges oriented in opposite circumferential directions, wherein in each case two zigzag foldings adjacent in the circumferential direction (U) are in each case folded mirror-symmetrically with respect to the imaginary mirror plane (17, 27) arranged between the respective two adjacent zigzag foldings and in the longitudinal direction of the tube, and
• arranging the compressed body (5, 55) within an inner free space (18, 28, 38, 48) of the folded covering (10, 20, 30, 40) or of the unfolded covering.

9. The method according to claim 8, **characterized in that** the unfolded tubular covering is produced by arranging two end portions of a flat covering one above the other and firmly connecting these superimposed end portions along the longitudinal direction of the tube.

## Patentansprüche

1. Implantat, das eine endovaskuläre Prothese, eine Herzklappe oder ein Implantat im neurovaskulären Bereich ist, umfassend einen Körper (5, 55), der eine umlaufende und durchbrochene Mantelfläche ausbildet, wobei der Körper (5, 55) einen komprimierten Zustand aufweist und dazu ausgebildet ist, in einen expandierten Zustand überzugehen, wobei die Mantelfläche im komprimierten Zustand des Körpers (5, 55) zumindest abschnittsweise mit einer schlauchförmigen Bedeckung (10, 20, 30, 40) bedeckt ist, **dadurch gekennzeichnet, dass** die Bedeckung (10, 20, 30, 40) entlang einer Umfangsrichtung (U) der schlauchförmigen Bedeckung in eine erste Zickzackfaltung (11, 21, 31, 41) und eine zweite Zickzackfaltung (12, 22, 23, 24, 32, 42) oder eine erste Zickzackfaltung (21), eine zweite Zickzackfaltung (22), eine dritte Zickzackfaltung (23) und eine vierte Zickzackfaltung (24) oder eine Anzahl von 2 x N Zickzackfaltungen gelegt ist, wobei N >= 3, wobei alle Zickzackfaltungen in der Umfangsrichtung (U) nebeneinander angeordnet sind und jede Zickzackfaltung drei in Umfangsrichtung verlaufende, übereinander liegende Lagen des Bedeckungsmaterials und zwei Faltkanten aufweist, die in entgegengesetzte Umfangsrichtungen orientiert sind, wobei jeweils zwei in der Umfangsrichtung (U) benachbarte Zickzackfaltungen jeweils spiegelsymmetrisch bezogen auf die zwischen den jeweiligen zwei benachbarten Zickzackfaltungen angeordnete gedachte Spiegelebene (17, 27) und in Längsrichtung des Schlauchs gefaltet sind.

2. Implantat nach Anspruch 1, **dadurch gekennzeichnet, dass** die Bedeckung einen Verbindungsabschnitt aufweist, in dem eine erste Lage des Bedeckungsmaterials und eine zweite Lage des Bedeckungsmaterials übereinander liegen und entlang der Längsrichtung der Bedeckung aneinander befestigt sind.

3. Implantat nach Anspruch 2, **dadurch gekennzeichnet, dass** der Verbindungsabschnitt in Umfangsrichtung benachbart zu dem Faltungsabschnitt der Bedeckung angeordnet ist, der die erste Zickzackfaltung (11) und die mindestens eine zweite Zickzackfaltung (12) umfasst, derart, dass der Verbindungsabschnitt und der Faltungsabschnitt nicht überlappen.

4. Implantat nach Anspruch 2, **dadurch gekennzeichnet, dass** der Verbindungsabschnitt mit mindestens einer Faltung überlappt, die durch die erste Zickzackfaltung (21, 31, 41) und/oder die mindestens eine zweite Zickzackfaltung (22, 23, 24, 32, 42) gebildet ist.

5. Implantat nach einem der Ansprüche 2 und 4, **dadurch gekennzeichnet, dass** der Verbindungsabschnitt bezogen auf die radiale Richtung der Bedeckung zumindest teilweise unterhalb der mindestens einen Faltung angeordnet ist, die durch die erste Zickzackfaltung (41) und/oder die mindestens eine zweite Zickzackfaltung (42) gebildet ist.

6. Implantat nach einem der Ansprüche 2 und 4, **dadurch gekennzeichnet, dass** der Verbindungsabschnitt bezogen auf die radiale Richtung der Bedeckung die mindestens eine Faltung, die durch die erste Zickzackfaltung (21, 31) und/oder die mindestens eine zweite Zickzackfaltung (22, 23, 24, 32) gebildet ist, zumindest teilweise außen umgibt.

7. Implantat nach Anspruch 1, **dadurch gekennzeichnet, dass** der Körper (5, 55) aus einer Vielzahl von miteinander verbundenen Streben (7, 57) zusammengesetzt ist, die im komprimierten Zustand eine im Wesentlichen hohlzylindrische Form ausbilden.

8. Verfahren zur Herstellung eines Implantats, das eine endovaskuläre Prothese, eine Herzklappe oder ein Implantat im neurovaskulären Bereich ist, nach einem der Ansprüche 1 bis 7, wobei das Verfahren die folgenden Schritte umfasst:
• Bereitstellen eines Körpers (5, 55) in dem komprimierten Zustand,
• Bereitstellen einer ungefalteten schlauchförmigen Bedeckung (10a), die eine vorgegebene Länge (L) in der Längsrichtung aufweist,
• Falten der Bedeckung derart, dass das Material entlang der Umfangsrichtung (U) in eine erste Zickzackfaltung (11, 21, 31, 41) und eine zweite Zickzackfaltung (12, 22, 23, 24, 32, 42) oder eine erste Zickzackfaltung (21), eine zweite Zickzackfaltung (22), eine dritte Zickzackfaltung (23) und eine vierte Zickzackfaltung (24) oder eine Anzahl von 2 x N Zickzackfaltungen gelegt wird, wobei N >= 3, wobei alle Zickzackfaltungen in der Umfangsrichtung (U) nebeneinander angeordnet sind und jede Zickzackfaltung drei in Umfangsrichtung verlaufende übereinander liegende Lagen des Bedeckungsmaterials und zwei Faltkanten aufweist, die in entgegengesetzte Umfangsrichtungen orientiert sind, wobei jeweils zwei in der Umfangsrichtung (U) benachbarte Zickzackfaltungen jeweils spiegelsymmetrisch bezogen auf die zwischen den jeweiligen zwei benachbarten Zickzackfaltungen angeordnete gedachte Spiegelebene (17, 27) und in Längsrichtung des Schlauchs gefaltet sind, und
• Anordnen des komprimierten Körpers (5, 55) innerhalb eines inneren Freiraums (18, 28, 38, 48) der gefalteten Bedeckung (10, 20, 30, 40) oder der ungefalteten Bedeckung.

9. Verfahren nach Anspruch 8, **dadurch gekennzeichnet, dass** die ungefaltete schlauchförmige Bedeckung durch Anordnen zweier Endabschnitte einer flachen Bedeckung übereinander und festes Verbinden dieser übereinander liegenden Endabschnitte entlang der Längsrichtung des Schlauchs hergestellt wird.

## Revendications

1. Implant qui est une prothèse endovasculaire, une valve cardiaque ou un implant dans le domaine neurovasculaire, comprenant un corps (5, 55) formant une surface latérale circonférentielle et perforée, dans lequel le corps (5, 55) présente un état comprimé et est configuré pour passer à un état expansé, dans lequel la surface latérale, à l'état comprimé du corps (5, 55), est recouverte au moins par portions d'un revêtement tubulaire (10, 20, 30, 40), **caractérisé en ce que** le revêtement (10, 20, 30, 40) est disposé le long d'une direction circonférentielle (U) du revêtement tubulaire en un premier pliage en zigzag (11, 21, 31, 41) et un deuxième pliage en zigzag (12, 22, 23, 24, 32, 42) ou en un premier pliage en zigzag (21), un deuxième pliage en zigzag (22), un troisième pliage en zigzag (23) et un quatrième pliage en zigzag (24) ou en un nombre de 2 x N pliages en zigzag, où N >= 3, dans lequel tous les pliages en zigzag sont disposés les uns à côté des autres dans la direction circonférentielle (U) et chaque pliage en zigzag présente trois couches superposées du matériau de revêtement, lesquelles s'étendent dans la direction circonférentielle, et deux arêtes de pli orientées dans des directions circonférentielles opposées, dans lequel, dans chaque cas, deux pliages en zigzag adjacents dans la direction circonférentielle (U) sont, dans chaque cas, pliés de manière symétrique en miroir par rapport au plan de miroir imaginaire (17, 27) disposé entre les deux pliages en zigzag adjacents respectifs et dans la direction longitudinale du tube.

2. Implant selon la revendication 1, **caractérisé en ce que** le revêtement comprend une portion de liaison dans laquelle une première couche du matériau de revêtement et une deuxième couche du matériau de revêtement sont superposées et fixées l'une à l'autre le long de la direction longitudinale du revêtement.

3. Implant selon la revendication 2, **caractérisé en ce que** la portion de liaison est disposée de manière adjacente, dans la direction circonférentielle, à la portion de pliage du revêtement comprenant le premier pliage en zigzag (11) et l'au moins un deuxième pliage en zigzag (12), de telle sorte que la portion de liaison et la portion de pliage ne se chevauchent pas.

4. Implant selon la revendication 2, **caractérisé en ce que** la portion de liaison chevauche au moins un pli formé par le premier pliage en zigzag (21, 31, 41) et/ou l'au moins un deuxième pliage en zigzag (22, 23, 24, 32, 42).

5. Implant selon l'une quelconque des revendications 2 et 4, **caractérisé en ce que** la portion de liaison est disposée au moins partiellement en dessous de l'au moins un pli formé par le premier pliage en zigzag (41) et/ou l'au moins un deuxième pliage en zigzag (42) par rapport à la direction radiale du revêtement.

6. Implant selon l'une quelconque des revendications 2 et 4, **caractérisé en ce que** la portion de liaison entoure extérieurement au moins partiellement l'au moins un pli formé par le premier pliage en zigzag (21, 31) et/ou l'au moins un deuxième pliage en zigzag (22, 23, 24, 32) par rapport à la direction radiale du revêtement.

7. Implant selon la revendication 1, **caractérisé en ce que** le corps (5, 55) est composé d'une pluralité d'entretoises (7, 57) reliées entre elles qui forment une forme sensiblement cylindrique creuse à l'état comprimé.

8. Procédé de production d'un implant qui est une prothèse endovasculaire, une valve cardiaque ou un implant dans le domaine neurovasculaire selon l'une quelconque des revendications 1 à 7, ledit procédé comprenant les étapes suivantes :
fournir un corps (5, 55) à l'état comprimé,
fournir un revêtement tubulaire déplié (10a) ayant une longueur prédéterminée (L) dans la direction longitudinale,
plier le revêtement de telle manière que le matériau est disposé le long de la direction circonférentielle (U) en un premier pliage en zigzag (11, 21, 31, 41) et un deuxième pliage en zigzag (12, 22, 23, 24, 32, 42) ou en un premier pliage en zigzag (21), un deuxième pliage en zigzag (22), un troisième pliage en zigzag (23) et un quatrième pliage en zigzag (24) ou en un nombre de 2 x N pliages en zigzag, où N >= 3, dans lequel tous les pliages en zigzag sont disposés les uns à côté des autres dans la direction circonférentielle (U) et chaque pliage en zigzag présente trois couches superposées du matériau de revêtement, lesquelles s'étendent dans la direction circonférentielle, et deux arêtes de pli orientées dans des directions circonférentielles opposées, dans lequel, dans chaque cas, deux pliages en zigzag adjacents dans la direction circonférentielle (U) sont, dans chaque cas, pliés de manière symétrique en miroir par rapport au plan de miroir imaginaire (17, 27) disposé entre les deux pliages en zigzag adjacents respectifs et dans la direction longitudinale du tube, et
disposer le corps comprimé (5, 55) à l'intérieur d'un espace libre intérieur (18, 28, 38, 48) du revêtement plié (10, 20, 30, 40) ou du revêtement déplié.

9. Procédé selon la revendication 8, **caractérisé en ce que** le revêtement tubulaire déplié est produit en disposant deux portions d'extrémité d'un revêtement plat l'une au-dessus de l'autre et en reliant fermement ces portions d'extrémité superposées le long de la direction longitudinale du tube.
